# EUROPEAN PATENT APPLICATION

(11) **EP 4 360 644 A1**
(43) Date of publication of application: **01.05.2024**
(21) Application number: 22828819.7
(22) Date of filing: 24.06.2022
(51) Int. Cl.: A61K 38/17, A61K 9/00, A61K 45/06, A61P 9/00, A61P 13/12

(54) **EFPEGLENATIDE TO REDUCE RISK OF DEVELOPING CARDIOVASCULAR DISEASE OR RENAL DYSFUNCTION**

(30) Priority: 25.06.2021 KR 20210083100
(71) Applicant: Hanmi Pharm. Co., Ltd., Hwaseong-si, Gyeonggi-do 18536 (KR)
(72) Inventor: BAEK, Seung Jae, Seoul 05545 (KR); HAN, Oak Pil, Seoul 05545 (KR)
(74) Representative: Brevalex
(86) International application number: PCT/KR2022/009027
(87) International publication number: WO 2022/270972

(57) **Abstract**

Provided are a use, a pharmaceutical composition, and a method for efpeglenatide for reducing a risk of developing a cardiovascular disease or renal dysfunction. The efpeglenatide may be used for cardiovascular or renal protection of patients having type 2 diabetes who have or are at risk of a cardiovascular disease or renal disease.

## Description

### Technical Field

It relates to uses, pharmaceutical compositions, and methods for efpeglenatide for reducing the risk of developing cardiovascular disease or renal dysfunction.

### Background Art

Efpeglenatide is the International Nonproprietary Name (INN) assigned by the World Health Organization (WHO) to a long-acting CA-exendin-4 derivative (LAPSCA-Exendin4). Efpeglenatide is a GLP-1 RA that is injected subcutaneously weekly and lowers glucose levels without causing hypoglycemia, and includes a modified exendin-4 molecule conjugated to an IgG4 Fc fragment (Patent Documents 1 and 2).

Diabetic patients have a three-fold increase in the incidence rate of cardiovascular (CV) outcome and a high incidence rate of renal outcome. The incidence rate of CV outcome increases according to duration of diabetes, HbA1c, urine albumin to creatinine ratio (UACR), renal dysfunction, prior CV disease, and other CV risk factors. In addition, similar factors increase the incidence rate of renal outcomes. Likewise, diabetes is known to increase cardiovascular disease and worsen renal function, but it is not well known whether controlling diabetes reduces the incidence rate of cardiovascular disease, and whether renal function is improved is only limited to some substances.

Four glucagon-like peptide 1 receptor agonists (GLP-1 RAs) based on human GLP-1 reduce cardiovascular outcomes in type 2 diabetes mellitus. However, the effect of an exendin-based GLP-1 RA on CV and renal outcomes in people at high risk of developing CV is uncertain. Additionally, the effect of exendin-based GLP-1 RA on CV and renal outcomes when combined with a sodium-glucose co-transporter-2 (SGLT2) inhibitor is unknown.
(Patent Document 1) WO 2008/082274 A1
(Patent Document 2) WO 2009/011544 A2

### Disclosure

### Technical Problem

Accordingly, inventors of the present disclosure completed the present disclosure by confirming through clinical trials that cardiovascular disease was reduced and renal function was improved when diabetes was controlled using efpeglenatide. Therefore, the present disclosure provides the following uses of efpeglenatide:
Provided is an efpeglenatide for preventing the development of cardiovascular disease or renal dysfunction in a patient, reducing the risk of developing the same, or delaying the progression thereof.

Provided is a pharmaceutical composition including efpeglenatide, wherein the pharmaceutical composition prevents the development of cardiovascular disease or renal dysfunction in a patient, or reduces the risk of development thereof.

Provided is a method of preventing the development of cardiovascular disease or renal dysfunction in a patient with type 2 diabetes mellitus, or reducing the risk of development thereof, the method including administering an effective amount of efpeglenatide to the patient.

Provided is a method of treating type 2 diabetes mellitus, the method including administering an effective amount of efpeglenatide to a patient, wherein the patient does not experience adverse effects associated with cardiovascular disease or renal dysfunction during treatment.

Provided is the use of efpeglenatide for use in the treatment of a patient with type 2 diabetes mellitus, wherein efpeglenatide prevents the development of cardiovascular disease or renal dysfunction in the patient, or reduces the risk of the development thereof.

### Technical Solution

One aspect provides an efpeglenatide for preventing the development of cardiovascular disease or renal dysfunction, reducing the risk of developing the same, or delaying the progression thereof in a patient.

Another aspect provides a pharmaceutical composition including efpeglenatide, wherein the pharmaceutical composition is for preventing the development of cardiovascular disease or renal dysfunction, or reducing the risk of developing the same, in a patient.

Another aspect provides a method of preventing the development of cardiovascular disease or renal dysfunction in a patient with type 2 diabetes mellitus, or reducing the risk of development thereof, including administering an effective amount of efpeglenatide to the patient.

Another aspect provides a method of treating type 2 diabetes mellitus, the method including administering an effective amount of efpeglenatide to a patient, wherein the patient does not experience adverse effects associated with cardiovascular disease or renal dysfunction during treatment.

Another aspect provides the use of efpeglenatide for use in the treatment of a patient with type 2 diabetes mellitus, wherein efpeglenatide prevents the development of cardiovascular disease or renal dysfunction in the patient, or reduces the risk of the development thereof.

As used herein, efpeglenatide is a type of insulinotropic peptide conjugate in which an exendin-4 derivative is linked to the immunoglobulin Fc region through a non-peptide polymer. The substances of these insulinotropic peptide conjugates (for example: efpeglenatide) and their medicinal uses are described in PCT International Publication No. WO 2008/082274 A1 and WO 2009/011544 A2, each of which is incorporated herein by reference.

In an Example, participants with type 2 diabetes mellitus who either (1) had prior cardiovascular (CV) disease, or (2) had an eGFR of 25 to 59.9 ml/min/1.73 m² and met one or more other CV risk factors, were subcutaneously administered efpeglenatide 4 mg or 6 mg, or placebo weekly. As a result, it was confirmed that the efpeglenatide administration group had statistically significant beneficial effects in cardiovascular protection and renal protection, including a 27 % decrease in major adverse CV events (MACE) and a 32 % decrease in composite kidney outcome compared to the placebo group. In other words, it was confirmed that weekly administration of 4 mg or 6 mg of efpeglenatide reduces the risk of cardiovascular or renal events in a patient with type 2 diabetes mellitus accompanied by cardiovascular or renal disease. Accordingly, efpeglenatide may be used in uses, pharmaceutical compositions, methods, etc. for preventing the development of cardiovascular disease or renal dysfunction in a patient, to reduce the risk of developing the same, or to delay the progression of the same.

### <Patient>

As used herein, a patient refers to an individual in need of preventing the development of, reducing the risk of developing, or delaying the progression of cardiovascular disease or renal dysfunction.

In an embodiment, the patient may be a patient with type 2 diabetes mellitus (T2DM).

In an embodiment, the patient may be a patient with non-alcoholic steatohepatitis (NASH).

In an embodiment, the patient may be a type 2 diabetes mellitus patient with an HbA1c greater than 7 %. An HbA1c of less than 5.7 % is normal, and an HbA1c of greater than 7 % may be diagnosed as type 2 diabetes mellitus.

In an embodiment, the patient may be a type 2 diabetes mellitus patient who has or is at risk for cardiovascular disease, renal disease, or both.

In an embodiment, the patient may be a type 2 diabetes mellitus patient with cardiovascular disease.

In an embodiment, the patient is an adult (for example, 18 years of age or older).

In an embodiment, the patient may be a type 2 diabetes mellitus patient with cardiovascular disease who is 18 years of age or older.

In an embodiment, the cardiovascular disease may be selected from coronary artery disease, stroke, and peripheral artery disease. The cardiovascular disease may be a previous cardiovascular disease or a current cardiovascular disease.

In an embodiment, the patient may be a type 2 diabetes mellitus patient 18 years of age or older with a cardiovascular disease selected from coronary artery disease, stroke, and peripheral artery disease. Therefore, the patient may be a type 2 diabetes mellitus patient who has or is at risk of having cardiovascular disease.

In an embodiment, the patient may be a type 2 diabetes mellitus patient with renal disease.

The renal disease may refer to impaired renal function, which may be assessed by estimated glomerular filtration rate (eGFR). The estimated glomerular filtration rate (eGFR) may be calculated by the MDRD equation using age, gender, race, and serum creatinine concentration, and details of the MDRD equation are provided in Examples herein. The eGFR may be used to assess renal function. An eGFR of 60 or more but less than 90 ml/min/1.73 m² is defined as renal damage and mild decrease in GFR, an eGFR of 30 or more but less than 60 ml/min/1.73 m² is defined as moderate decrease in GFR, an eGFR of 15 or more but less than 30 ml/min/1.73 m² is defined as severe decrease in GFR, and an eGFR of less than 15 ml/min/1.73 m² or dialysis is defined as renal failure.

In an embodiment, the patient may have a moderate or severe decrease in GFR.

In an embodiment, the patient may have an eGFR of 25 or more but less than 60 ml/min/1.73 m².

In an embodiment, the patient may have an eGFR of 25 to 59.9 ml/min/1.73 m².

In an embodiment, the patient may have one or more cardiovascular disease risk factors. Where the patient has one or more cardiovascular disease risk factors, the patient may be defined as having or at risk of having a cardiovascular disease. The cardiovascular disease risk factor may be selected from (a) to (f) below:
(a) A body mass index (BMI) of 35 kg/m² or more;
(b) LDL greater than 3.36 mmol/L and HDL less than 1.03 mmol/L for men or less than 1.29 mmol/L for women within last 6 months while taking statins;
(c) smoking;
(d) urine albumin-to-creatinine ratio (UACR) of 3 mg/mmol or more;
(e) systolic blood pressure of 140 mmHg or more and diastolic blood pressure of 90 mmHg or more, and taking antihypertensive medication; and
(f) first degree coronary artery disease at age less than 55 years for men or age less than 65 years for women.

In an embodiment, the patient may be a type 2 diabetes mellitus patient with an eGFR of 25 to 59.9 ml/min/1.73 m² and one or more cardiovascular disease risk factors as defined above.

In an embodiment, the patient may be a male type 2 diabetes mellitus patient 50 years of age or older or a female type 2 diabetes mellitus patient 55 years of age or older with an eGFR of 25 to 59.9 ml/min/1.73 m² and one or more cardiovascular disease risk factors as defined above. Therefore, the patient may be a type 2 diabetes mellitus patient who has or is at risk of having both renal disease and cardiovascular disease.

In an embodiment, the patient is a type 2 diabetes mellitus patient and may be (1) 18 years of age or older with prior coronary artery disease, stroke, or peripheral artery disease; or (2) a male 50 years of age or older or a female 55 years of age or older with an eGFR of 25 to 59.9 ml/min/1.73 m² and one or more of cardiovascular disease risk factors (a) to (f) as defined above. However, the patient is not necessarily limited to a patient with type 2 diabetes mellitus and may include any patient who has or is at risk of having cardiovascular disease and/or renal disease.

In an embodiment, the patient may be, but is not limited to, a patient without any of the following: gastroparesis, uncontrolled reflux, prolonged nausea or vomiting, severe retinal disease, and pancreatitis.

In an embodiment, the patient may be a type 2 diabetes mellitus patient who has not used a glucagon-like peptide-1 (GLP-1) receptor agonist or dipeptidyl peptidase-4 (DPP-4) inhibitor within the last 3 months. However, this is intended to minimize the effects of conventional GLP-1 receptor agonists or DPP-4 inhibitors and is not a requirement for these conditions to be met.

### <Administration route, dosage and usage>

The efpeglenatide may be administered to a patient according to an appropriate administration route, dose, and usage in order to prevent the development of cardiovascular disease or renal dysfunction, reduce the risk of development of cardiovascular disease or renal dysfunction, or delay the progression of cardiovascular disease or renal dysfunction in the patient.

The efpeglenatide may be administered to a patient by any appropriate route of administration. In an embodiment, the efpeglenatide may be administered subcutaneously. The method for the subcutaneous administration may use any appropriate method known in the art. For example, the subcutaneous administration may be performed by a syringe pre-filled with efpeglenatide at an appropriate administration dosage, but is not limited thereto.

The efpeglenatide may be administered to the patient in an effective amount. The effective amount may be a pharmaceutically effective amount. The effective amount may refer to an amount that may exert the intended therapeutic effect of efpeglenatide.

In an embodiment, the effective amount of efpeglenatide may be administered weekly.

In an embodiment, the effective amount of efpeglenatide may be 4 mg or 6 mg weekly. Therefore, the efpeglenatide may be administered weekly at 4 mg or 6 mg.

In an embodiment, the efpeglenatide may be administered weekly at 2 mg for 4 weeks, and then administered weekly at 4 mg.

In an embodiment, the efpeglenatide may be administered weekly at 2 mg for 4 weeks, then administered weekly at 4 mg for 4 weeks, and then administered weekly at 6 mg.

The efpeglenatide may be administered at least once or more, weekly for a period of at least 4 weeks or more, at least 3 months or more, at least 6 months or more, or at least 1 year or more. The duration of treatment may be extended until the desired therapeutic effect may be achieved.

### <Use related to cardiovascular disease or renal dysfunction>

In the composition, method and/or use using efpeglenatide, the efpeglenatide is provided for preventing the development of, reducing the risk of development of, or delaying the progression of cardiovascular disease or renal dysfunction in a patient.

The term "preventing the development of cardiovascular disease or renal dysfunction" may refer to preventing the development of new or worsening of cardiovascular disease or renal dysfunction in a patient by administration of efpeglenatide.

The term "reducing the risk of developing cardiovascular disease or renal dysfunction" may refer to administration of efpeglenatide reducing the risk of developing new or worsening cardiovascular disease or renal dysfunction in a patient compared to administration of placebo.

The term "delaying the progression of cardiovascular disease or renal dysfunction" may refer to delaying the development of new cardiovascular disease or renal dysfunction in a patient, reducing the rate of progression of pre-existing cardiovascular disease or renal dysfunction, or reducing the rate at which cardiovascular disease or renal dysfunction worsens by administration of efpeglenatide.

Accordingly, the efpeglenatide may be provided to use to treat type 2 diabetes mellitus in a patient at risk of developing cardiovascular disease or renal dysfunction. When used to treat a patient with type 2 diabetes mellitus, efpeglenatide may reduce the risk of developing cardiovascular disease or renal dysfunction in the patient compared to placebo or other type 2 diabetes mellitus therapeutic agents. Accordingly, the patient may not experience cardiovascular diseases (for example: MACE) or renal events (for example: adverse effects associated with renal dysfunction) during treatment of the disease with efpeglenatide.

Meanwhile, the efpeglenatide may be provided for the use of preventing the development of cardiovascular disease or renal dysfunction, reducing the risk of development of cardiovascular disease or renal dysfunction, or delaying the progression of cardiovascular disease or renal dysfunction in an individual in need of treatment by administration of efpeglenatide. In other words, efpeglenatide may be provided for use in cardiovascular or renal protection. The individual in need of treatment by administration of efpeglenatide may be a type 2 diabetes mellitus patient or may not be a type 2 diabetes mellitus patient. If not a type 2 diabetes mellitus patient, the individual may be a patient that has a disease that may be treated by administration of efpeglenatide.

The cardiovascular disease or renal dysfunction may be determined by whether the patient experiences an outcome.

In an embodiment, the cardiovascular disease may be a primary outcome. The cardiovascular disease may be a major adverse CV event (MACE). The MACE may be selected from non-fatal myocardial infarction, non-fatal stroke, and death from cardiovascular or unspecified causes, as such terms are well known in the art. The efpeglenatide may reduce the risk of developing MACE by 20 % or more, for example 27 %, compared to placebo. For example, the efpeglenatide administration group may have a MACE incidence of 3.9 per 100 people per year, while the placebo group may have a MACE incidence of 5.3 per 100 people per year.

In an embodiment, the renal dysfunction may be a secondary outcome. The renal dysfunction may be a composite kidney outcome. The renal dysfunction may include one or more composite kidney outcomes selected from (a) to (d) below:
(a) Macroalbuminuria (defined as a UACR greater than 33.9 mg/mmol and 30 % or more above baseline);
(b) a sustained decrease in eGFR of 40 % or more for 30 days or more;
(c) renal replacement therapy for 90 days or more; and
(d) eGFR less than 15 ml/min/1.73 m² for 30 days or more continuously.

As used herein, "baseline" refers to a value measured for each individual patient prior to administration of efpeglenatide. Accordingly, this baseline value may vary from one individual patient to another.

Compared to placebo, efpeglenatide may reduce the risk of developing the above-defined composite kidney outcome by 30 % or more, for example 32 %. Specifically, efpeglenatide may prevent the development of, reduce the risk of, or delay the progression of macroalbuminuria in a patient. The efpeglenatide may prevent, reduce the risk of, or delay the progression of a sustained decline in eGFR of 40 % or more for 30 days or more in a patient. The efpeglenatide may reduce the risk of renal replacement therapy in a patient. In an embodiment, the efpeglenatide may prevent, reduce the risk of, or delay the progression of a sustained decline in eGFR to less than 15 ml/min/1.73 m² for 30 days or more in a patient.

In an embodiment, the cardiovascular disease or renal dysfunction may be an other outcome. The cardiovascular diseases may include MACE, coronary revascularization, and hospitalization for unstable angina. Therefore, the efpeglenatide may prevent the development of, reduce the risk of developing, or delay the progression of selected cardiovascular diseases, including MACE, coronary revascularization, and hospitalization for unstable angina.

In an embodiment, the efpeglenatide may reduce the risk of development of death from MACE or non-cardiovascular death compared to placebo.

### <Other therapeutic substance>

The efpeglenatide may be administered in combination with any one or more other therapeutic substances. Even when used in combination with other therapeutic substances, efpeglenatide may be effective in preventing the development of cardiovascular disease or renal dysfunction in patients, reduce the risk of its development, or delay its progression.

The other therapeutic substance may be selected from, but is not limited to, a hypoglycemic drug, cardioprotective drug, anti-diabetic agent, anti-obesity agent, anti-hyperlipidemic agent, anti-atherosclerotic agent, anti-hypertensive agent, anti-platelet agent, anti-thrombotic agent and anti-coagulant agent. The other therapeutic substances are not particularly limited as long as they are known in the art, but it is preferred to use substances that do not significantly affect the efficacy of efpeglenatide.

In an embodiment, the other therapeutic substance may be selected from a sodium glucose co-transporter 2 (SGLT2) inhibitor and metformin. For example, the other therapeutic substance may be an SGLT2 inhibitor.

The other therapeutic substance may be administered concurrently, sequentially, or separately from efpeglenatide. The dosage and route of administration of the other therapeutic substance may be selected as appropriate for the substance.

### <Pharmaceutical Composition>

A pharmaceutical composition according to an aspect includes an efpeglenatide and may be for preventing the development of, or reducing the risk of developing, cardiovascular disease or renal dysfunction in a patient.

The pharmaceutical composition may be for preventing, treating, or improving cardiovascular disease or renal dysfunction.

The pharmaceutical composition may be for treating type 2 diabetes mellitus.

The pharmaceutical composition may be for preventing the development of cardiovascular disease or renal dysfunction, reducing the risk of development of cardiovascular disease or renal dysfunction, or delaying progression of cardiovascular disease or renal dysfunction in a patient with type 2 diabetes mellitus.

The pharmaceutical composition may be for cardiovascular or renal protection in a patient with type 2 diabetes mellitus.

The efpeglenatide may be in any pharmaceutically acceptable form.

"Pharmaceutically acceptable" refers to an amount sufficient to produce a therapeutic effect and without causing adverse effects, and may be readily determined by a person skilled in the art according to factors well known in the medical field, such as the type of disease, the patient's age, weight, health, gender, the patient's sensitivity to the drug, the route of administration, the method of administration, the number of doses, the duration of treatment, and the combination or concomitant drugs used.

The pharmaceutical composition may be formulated in an appropriate form for administering efpeglenatide to a patient. Additionally, the pharmaceutical composition may further include a pharmaceutically acceptable carrier, excipient, or diluent. Carriers, excipients and diluents suitable for formulation may be appropriately selected by a person skilled in the art from any known substance.

In an embodiment, the pharmaceutical composition may include a pharmaceutically effective amount of efpeglenatide and excipients.

In an embodiment, the pharmaceutical composition may be a liquid formulation. Liquid formulations of efpeglenatide may refer, for example and without limitation, to WO 2014/017845, which is incorporated herein by reference.

In an embodiment, the pharmaceutical composition may be an injectable formulation. The pharmaceutical composition may be an injectable formulation for subcutaneous administration. When the pharmaceutical composition is an injectable formulation, a mixture of any ingredients selected from buffers, stabilizers, isotonic agents, preservatives, solubilizers, etc. may be used.

### <Method of use>

According to an aspect, provided is a method of preventing the development of, or reducing the risk of developing, cardiovascular disease or renal dysfunction in a patient with type 2 diabetes mellitus, including administering an effective amount of efpeglenatide to the patient.

According to an aspect, provided is a method of treating type 2 diabetes mellitus, the method including administering an effective amount of efpeglenatide to a patient, wherein the patient does not experience adverse effects associated with cardiovascular disease or renal dysfunction during treatment.

According to an aspect, provided is a method of protecting the cardiovascular or renal system during treatment of type 2 diabetes mellitus, including administering an effective amount of efpeglenatide to a patient.

The above aspects may further include identifying the patient prior to administering the efpeglenatide. The patient may be an individual in need of administration of efpeglenatide, an individual in need of reducing the risk of cardiovascular disease or renal dysfunction by administration of efpeglenatide, or an individual suitable for treatment using efpeglenatide. The patient may be a type 2 diabetes mellitus patient who has or is at risk of having cardiovascular disease, renal disease, or both.

In an embodiment, the method may further include, prior to administering efpeglenatide, identifying the patient as having any of the following:
(1) A type 2 diabetes mellitus patient with coronary artery disease, stroke, or peripheral artery disease; or
(2) a type 2 diabetes mellitus patient who has an eGFR of 25 to 59.9 ml/min/1.73 m² and meets one or more of the following cardiovascular disease risk factors (a) to (f):
   (a) A body mass index (BMI) of 35 kg/m² or more;
   (b) LDL greater than 3.36 mmol/L and HDL less than 1.03 mmol/L for men or less than 1.29 mmol/L for women within last 6 months while taking statins;
   (c) smoking;
   (d) urine albumin-to-creatinine ratio (UACR) of 3 mg/mmol or more;
   (e) systolic blood pressure of 140 mmHg or more and diastolic blood pressure of 90 mmHg or more, and taking antihypertensive medication; and
   (f) first degree coronary artery disease at age less than 55 years for men or age less than 65 years for women.

### <Treatment use>

According to an aspect, provided is the use of efpeglenatide for use in the treatment of a patient with type 2 diabetes mellitus, wherein efpeglenatide prevents the development of cardiovascular disease or renal dysfunction in the patient, or reduces the risk of the development thereof.

According to an aspect, provided is a use of efpeglenatide for cardiovascular or renal protection during treatment of a patient with type 2 diabetes mellitus.

Redundant content is omitted in consideration of the complexity of the present specification, and terms not otherwise defined in this specification have meanings commonly used in the technical field to which the present disclosure pertains.

### Advantageous Effects

Efpeglenatide according to an aspect may reduce the risk of developing cardiovascular or renal events. Therefore, efpeglenatide may be used to provide cardiovascular or renal protection in a type 2 diabetes mellitus patient who has or is at risk of having cardiovascular disease or renal disease. Additionally, efpeglenatide may achieve the above effects in combination with other medications, such as a SGLT2 inhibitor.

### Description of Drawings

FIGS. 1 to 4 are graphs of key cardiovascular and renal outcomes.
FIG. 1 illustrates the cumulative risk of the primary outcome MACE.
FIG. 2 illustrates the cumulative risk of three pre-specified secondary outcomes (MACE, coronary revascularization, or hospitalization for unstable angina).
FIG. 3 illustrates the cumulative risk of a secondary composite kidney outcome. The composite kidney outcome is defined as macroalbuminuria; a decrease in eGFR of 40 % or more for 30 days or more; renal replacement therapy for 90 days or more; or eGFR of less than 15 ml/min/1.73 m² for 30 days or more.
FIG. 4 illustrates the cumulative risk of the secondary outcome MACE or non-CV death.
FIG. 5 and FIG. 6 illustrate pre-specified subgroup analyses for the primary efficacy outcome.

### Mode for Invention

The present disclosure will be described in more detail below with reference to embodiments. However, these embodiments are intended to illustrate the present disclosure by way of example and the scope of the invention is not limited to these embodiments.

### Example

Efpeglenatide is a weekly subcutaneously injected GLP-1 RA that lowers glucose levels without causing hypoglycemia, and includes a modified exendin-4 molecule conjugated to an IgG4 Fc fragment. Because efpeglenatide is well tolerated and has the same mechanism of action as the human GLP-1-based GLP-1 RA, which reduces CV outcome, it was hypothesized that efpeglenatide may have CV and renal benefits in diabetic patients with CV and/or renal disease, and this hypothesis was tested in a clinical trial.

### 1. Method

### Participant

This clinical trial was an international randomized controlled trial conducted in 344 locations in 28 countries. Participants included patients with type 2 diabetes mellitus (T2DM) and an HbA1c of greater than 7 % who were either (1) 18 years of age or older with prior coronary artery disease, stroke, or peripheral artery disease; or (2) a male 50 years of age or older or a female 55 years of age or older with an estimated glomerular filtration rate (eGFR) of 25 to 59.9 ml/min/1.73 m² and met one or more of the following cardiovascular disease risk factors (a) to (f):

### <Cardiovascular disease risk factor>

(a) A body mass index (BMI) of 35 kg/m² or more;
(b) LDL greater than 3.36 mmol/L, HDL (for men) less than 1.03 mmol/L, or HDL (for women) less than 1.29 mmol/L within last 6 months while taking statins;
(c) smoking;
(d) urine albumin-to-creatinine ratio (UACR) of 3 mg/mmol or more;
(e) systolic blood pressure of 140 mmHg or more and diastolic blood pressure of 90 mmHg or more (while taking antihypertensive medication);
(f) first degree coronary artery disease at age less than 55 years (for men) or 65 years (for women).

The key exclusion criteria were gastroparesis, uncontrolled reflux, prolonged nausea or vomiting, severe retinal disease, pancreatitis, or use of GLP-1 receptor agonists or DPP-4 inhibitors within the previous 3 months.

The clinical trial was approved by research ethics committees at all sites, and all participants provided written informed consent.

### Randomization and masking

Eligible participants were randomly assigned in a 1:1:1 ratio to the 4 mg efpeglenatide administration group, the 6 mg efpeglenatide administration group, and the placebo group. The route of administration was self-administer subcutaneous injection, and the dosage and cycle of administration were as follows:
(1) 4 mg efpeglenatide administration group: administered weekly at 2 mg for 4 weeks, and then administered weekly at 4 mg;
(2) 6 mg efpeglenatide administration group: administered weekly at 2 mg for 4 weeks, then administered weekly at 4 mg for 4 weeks, and then administered weekly at 6 mg;
(3) Placebo group: placebo administration.

Study medications were provided in masked, identically appearing prefilled syringes. Randomization was performed using an interactive web response system (IWRS) stratified by sodium glucose co-transporter 2 (SGLT2) inhibitor use to minimize between-group differences in therapy with these cardioprotective drugs. Only the Data Monitoring Committee (DMC) had access to the unmasked data until the database was locked.

### Study procedures

Study medication was added to pre-randomization therapy. If baseline HbA1c was less than 7.5 %, the investigators could reduce the dose of any insulin, sulfonylurea, or meglitinide to minimize hypoglycemia. Subsequently, hypoglycemic drugs remained unchanged for the first 12 weeks, after which all medications except GLP-1 RA or DPP-4 inhibitors may be added. Post-randomization visits occurred at week 12, week 24, and every 24 weeks with interim telephone visits. Those who missed up to 2 doses of the study medication were encouraged to restart their usual dose unless there was a contraindication. If three or more consecutive doses were missed, bilateral masked titration was restarted. Unless consent was revoked, participants were followed until the end of the trial, regardless of medication adherence. Participants who remained on study medication at the end of the trial discontinued the medication and were followed up 6 weeks later. The clinical trial was terminated early before any of the 330 pre-specified events developed for reasons of sponsor management rather than drug safety or other reasons.

### Outcome

The primary outcome was a major adverse CV event (MACE). MACE was defined as non-fatal myocardial infarction, non-fatal stroke, or death from cardiovascular or unspecified causes.

The secondary outcome included an expanded MACE (which includes coronary revascularization or hospitalization for unstable angina in addition to MACE), and a composite kidney outcome that includes (a) to (d) below:

### <composite kidney outcome>

(a) Macroalbuminuria (i.e., urine albumin-to-creatinine ratio (UACR) greater than 300 mg/g or 33.9 mg/mmol and a UACR 30 % or more above baseline);
(b) a sustained decrease in eGFR of 40 % or more for 30 days or more;
(c) renal replacement therapy for 90 days or more;
(d) eGFR less than 15 ml/min/1.73 m² for 30 days or more continuously.

Exploratory endpoint (outcome) included components of the expanded MACE and renal outcomes, death from any cause, heart failure hospitalization, HbA1c, vital signs, weight, anti-efpeglenatide antibody, pancreatic enzyme, and other laboratory tests.

Prior to unblinding, three additional composite outcomes were defined:
a) MACE or non-CV death;
b) a renal function outcome defined as a 40 % or more decline in eGFR for 30 days or more, or end-stage renal disease (dialysis for 90 days or more, renal transplant, or eGFR less than 15 ml/min/1.73 m² for 30 days or more), or death from any cause; or
c) MACE, non-CV death, heart failure hospitalization, or renal function outcome.

An independent Clinical Endpoint Committee masked to treatment allocation adjudicated all deaths, myocardial infarction, strokes, hospitalization for unstable angina or heart failure, and pancreatic events. Spontaneouly reported and pre-specified adverse events were collected at each visit.

### Statistical analysis

In accordance with the 2008 FDA regulatory guidelines, to prove the non-inferiority of efpeglenatide compared to placebo for the MACE outcome, at the time, the limit of non-inferiority was set at 1.3, the power was set at 90 %, and it was decided to follow up to 4,000 participants for 3 years. The assumption used at the time was that the annual incidence of MACE was 3.7 % for SGLT2 users and 4.4 % for non-users, and it was predicted that 27 % of the entire subjects would take SGLT2. The annual drug discontinuation rate was calculated as 2 %, and the two-sided type 1 error was calculated as 5 %.

All analyzes were performed according to the statistical analysis plan established before unblinding, and all outcomes that developed between randomization and the last evaluation of all participants were counted using the intention-to-treat principle. Participants were censored as of the last date of follow-up for CV outcomes or death, and on the last day that renal outcome status was available for renal outcomes. Continuous variables were summarized as mean and standard deviation, or median and interquartile range, and categorical variables were summarized as numbers and percentages. Changes in continuous variables over time were analyzed using a mixed-effects model for repeated measures (MMRM) using restricted maximum likelihood, with baseline value as a covariate, participant as a random effect, and fixted effects for region (North America, Latin America, Europe, Other), three SGLT2 randomization strata, treatment group, visit timing, and treatment-by-visit interaction. Incidence rates for the first occurrence of a particular composite outcome, and the frequency of the total burden of a single or composite outcome were summarized as number per 100 person-years of follow-up for that outcome. Kaplan Meier curves were used to display cumulative risk, and Cox proportional hazards models adjusted for region, and the three SGLT2 randomization strata were used to estimate hazard ratios and 95 % confidence intervals for the effect of efpeglenatide (both doses combined) on the primary and secondary outcomes. The proportional means model was used to estimate the HR and 95 % Cl for total events, including recurrent events. The P value for non-inferiority was one-sided and the P value for superiority was 2-sided.

The effect of efpeglenatide within subgroups was assessed by including the subgroups and interaction terms in the Cox proportional hazards models. Exploratory shrinkage estimates of therapeutic effects within subgroups with two or more categories along with 95 % confidence intervals were estimated using Bayesian hierarchical modeling.

If non-inferiority was demonstrated for the primary outcome, additional outcomes were evaluated hierarchically until superiority was not significant: MACE; expanded CV composite; composite kidney outcome; MACE or non-CV death, new renal outcome, and MACE, non-CV death, heart failure, or new renal outcome.

Sample size was calculated using the PASS 13 software (NCSS, LLC, Kaysville, UT), shrinkage analysis was performed using the "bayesmeta" package in R version 3.5.1 (R Foundation for Statistical Computing), and all other statistical analyzes were performed using SAS version 9.4.

### Method summary

The randomized controlled trial, conducted at 344 sites in 28 countries, recruited patient with type 2 diabetes mellitus who either (1) had a prior CV disease or (2) had an eGFR of 25 to 59.9 ml/min/1.73 m² and met one or more other CV risk factors. Participants were randomly assigned to receive weekly subcutaneous efpeglenatide 4 mg or 6 mg, or placebo, with stratification by SGLT2 inhibitors use. The primary outcome was first MACE, defined as non-fatal myocardial infarction, non-fatal stroke, or death from cardiovascular or unspecified cause.

### 2. Result

A total of 5732 test subjects were screened between May 11, 2018 and April 25, 2019. Eligibility criteria were satisfied by 4076 participants who were then randomly assigned to efpeglenatide 4 mg administration group (subject: 1359 people), efpeglenatide 6 mg administration group (subject: 1358 people), or placebo group (subject: 1359 people). Follow-up ended on December 10, 2020, with a total of 7395.4 person-years and a median (IQR) of 1.81 years (1.69, 1.98). At the time, the proportion of patients who underwent MACE, the primary outcome, was 3941/4076 (96.7 %), and the vital status rate was 4073/4076 (99.9 %). Participants assigned to efpeglenatide or placebo were exposed to the study medication for 88.9 % and 91.1 % of their maximum follow-up time, respectively.

### Baseline characteristics

The baseline characteristics of the selected test subjects are shown in Table 1 below.

**[Table 1]**

| **Characteristic** | | **Both groups** | **Efpeglenatide administration group (4/6 mg)** | **Placebo group (Placebo)** |
|---|---|---|---|---|
| Number (N) | | 4076 | 2717 | 1359 |
| Age (Years) | | 64.5 (8.2) | 64.6 (8.2) | 64.4 (8.3) |
| Female | | 1344 (33.0) | 925 (34.0) | 419 (30.8) |
| Region: | Canada/US | 1079 (26.5) | 728 (26.8) | 351 (25.8) |
| | Mexico/South America | 924 (22.7) | 605 (22.3) | 319 (23.5) |
| | Europe | 1285 (31.5) | 862 (31.7) | 423 (31.1) |
| | Other | 788 (19.3) | 522 (19.2) | 266 (19.6) |
| White ancestry | | 3534 (86.7) | 2372 (87.3) | 1162 (85.5) |
| Diabetes mellitus duration | | 15.4 (8.8) | 15.6 (8.8) | 15.1 (8.7) |
| Current tobacco use | | 633 (15.5) | 427 (15.7) | 206 (15.2) |
| Prior cardiovascular disease (CVD)^{a} | | 3650 (89.6) | 2420 (89.1) | 1230 (90.5) |
| eGFR <60 ml/min/1.73m^{2b} | | 1287 (31.6) | 863 (31.8) | 424 (31.2) |
| Prior CVD & eGFR <60 ml/min/1.73m² | | 888 (21.8) | 585 (21.5) | 303 (22.3) |
| Prior heart failure | | 737 (18.1) | 487 (17.9) | 250 (18.4) |
| Prior hypertension | | 3722 (91.3) | 2484 (91.4) | 1238 (91.1) |
| Prior diabetic retinopathy^{c} | | 1342 (32.9) | 912 (33.6) | 430 (31.6) |
| N-Albuminuria (%)^{d} | | 1977 (48.5) | 1319 (48.5) | 658 (48.4) |
| Body mass index (kg/m²) | | 32.7 (6.2) | 32.9 (6.2) | 32.4 (6.0) |
| Heart rate (beats/min) | | 72.8 (10.6) | 72.8 (10.6) | 72.8 (10.7) |
| Systolic BP | | 134.9 (15.5) | 135.1 (15.5) | 134.4 (15.6) |
| Diastolic BP | 76.7 (9.7) | 76.8 (9.7) | 76.6 (9.8) | |
| HbA1c (%) | 8.91 (1.48) | 8.90 (1.46) | 8.94 (1.52) | |
| eGFR (ml/min/1.73m²)^{b} | 72.4 (22.4) | 72.2 (21.9) | 72.9 (23.3) | |
| Median albumin/creatine (mg/mmol) | 3.2 (1.1 - 12.9) | 3.2 (1.0 - 13.5) | 3.2 (1.1 - 12.0) | |
| Cholesterol (mmol/L) | 4.21 (1.23) | 4.21 (1.24) | 4.21 (1.22) | |
| LDL Cholesterol (mmol/L) | 2.07 (0.98) | 2.07 (0.98) | 2.08 (0.97) | |
| HDL Cholesterol (mmol/L) | 1.11 (0.31) | 1.12 (0.31) | 1.10 (0.31) | |
| Median Triglycerides (mmol/L) | 1.91 (1.37 - 2.75) | 1.90 (1.36 - 2.74) | 1.93 (1.40 - 2.75) | |
| Any insulin | 2560 (62.8) | 1720 (63.3) | 840 (61.8) | |
| Metformin | 2985 (73.2) | 1993 (73.4) | 992 (73.0) | |
| Sulfonylureas | 1036 (25.4) | 695 (25.6) | 341 (25.1) | |
| SGLT2 Inhibitor | 618 (15.2) | 412 (15.2) | 206 (15.2) | |
| No glucose-lowering drug | 85 (2.1) | 57 (2.1) | 28 (2.1) | |
| ACE-I or ARB or ARNi | 3262 (80.0) | 2177 (80.1) | 1085 (79.8) | |
| Beta Blocker | 2670 (65.5) | 1795 (66.1) | 875 (64.4) | |
| Statin | 3294 (80.8) | 2202 (81.1) | 1092 (80.4) | |
| Fibrate | 350 (8.6) | 233 (8.6) | 117 (8.6) | |
| Acetylsalicylic acid | 2768 (67.9) | 1855 (68.3) | 913 (67.2) | |
| Other anti-platelet drugs | 1049 (25.7) | 705 (26.0) | 344 (25.3) | |
| eGFR: estimated glomerular filtration rate; | | | | |
| ACE-I: angiotensin converting enzyme inhibitor; | | | | |
| ARB: angiotensin receptor blocker; | | | | |
| ARNI: angiotensin receptor-neprilysin inhibitor; | | | | |
| SGLT2: sodium-glucose co-transporter 2; | | | | |
| Expressed as mean (SD), median (IQR), or N (%); | | | | |
| ^{a} Coronary artery disease (i.e., prior myocardial infarction, stenosis of 50 % or more in the left aorta or 2 or more other coronary arteries, revascularization of either 2 or more coronary arteries or 1 coronary artery with stenosis of 50 % or morein another, or | | | | |
| stenosis of 50 % or more in 1 coronary artery with either a non-invasive test demonstrating ischemia or an unstable angina hospitalization in the prior 12 months); stroke, or peripheral arterial disease (i.e., limb angioplasty, peripheral artery stenting or bypass, limb or foot amputation due to circulatory insufficiency, ankle-brachial index less than 0.9, or angiographic evidence); | | | | |
| ^{b} eGFR by the 4-variable MDRD formula: 175 x [serum creatinine (µmol/L)/88.4]-1.154 x age (years)-0.203 x 1.212 (if Black) x 0.742 (if female); | | | | |
| ^{c} Self-report or vitrectomy, laser therapy, or ocular injections; | | | | |
| ^{d} albumin/creatinine ≥ 30 mg/g or 3.39 mg/mmol. | | | | |

As shown in Table 1, the recruited participants had a mean age of 64.5 (8.2) years, 1954 (47.9 %) were less than 65 years of age, and 1344 (33.0 %) were female. 3650 (89.6 %) had prior CV disease, defined as coronary artery disease, cerebrovascular disease, or peripheral artery disease; 1287 (31.6 %) had low eGFR (less than 60 ml/min/1.73 m²); 888 (21.8 %) had both CVD and low eGFR; and 618 (15.2 %) were using SGLT2i at baseline. A similar number of participants assigned to efpeglenatide or placebo were taking various glucose-lowering or cardioprotective drugs at baseline. However, at the last study visit, more participants assigned to placebo were taking a DPP-4 inhibitor (1.9 % vs. 0.9 %, p=0.005) and/or an SGLT2 inhibitor (21.2 % vs. 17.5 %, p=0.004).

### Trial outcomes

The adjudicated first events are shown in Table 2.

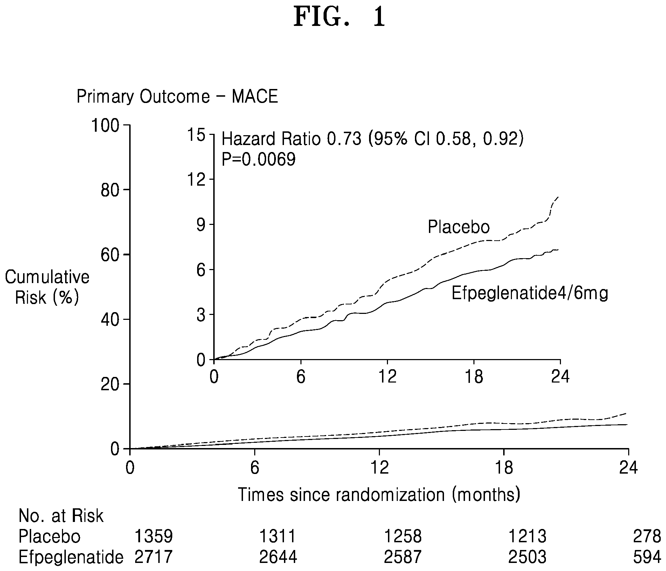

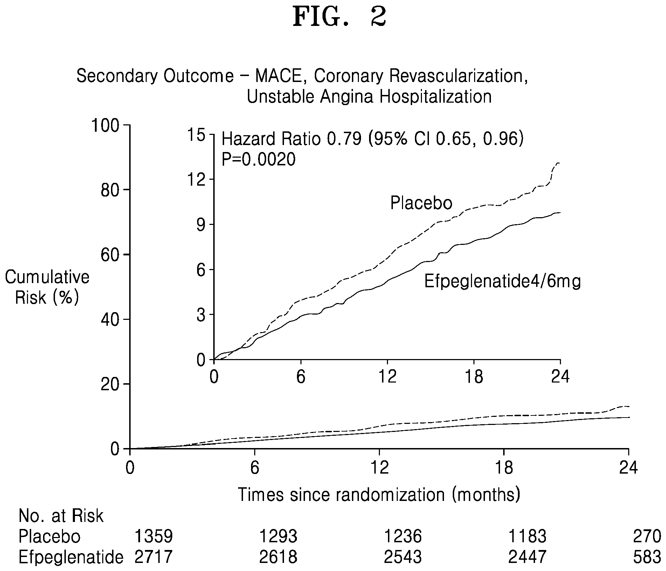

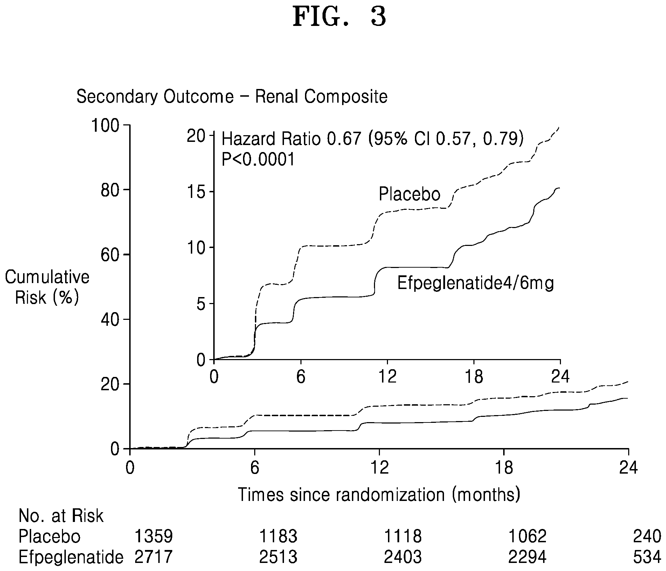

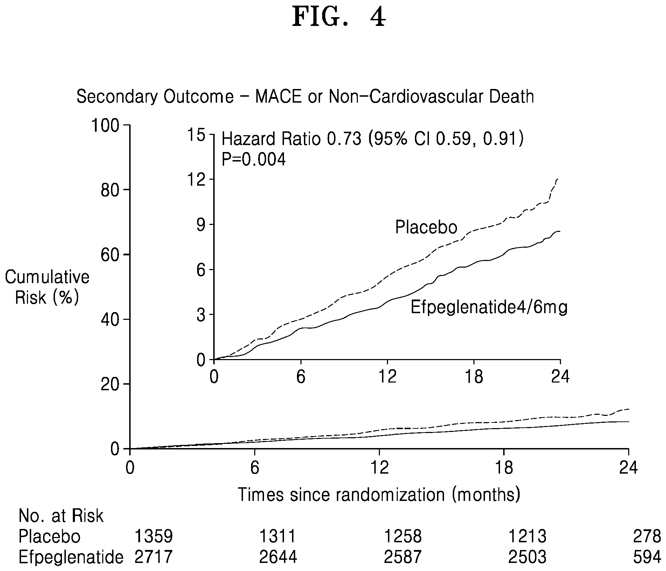

FIG. 1 to FIG. 4 are graphs of key cardiovascular and renal outcomes.

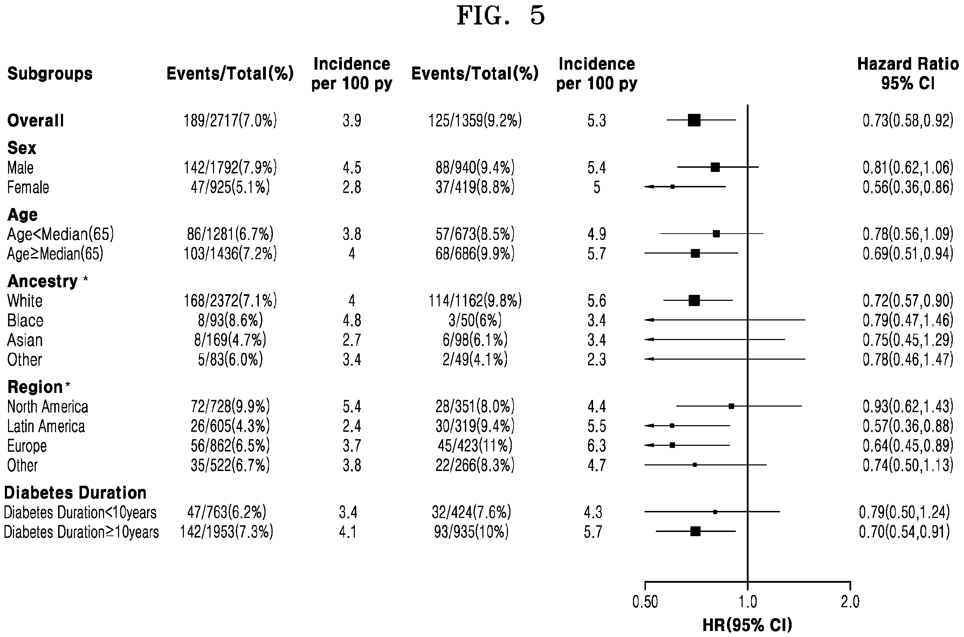

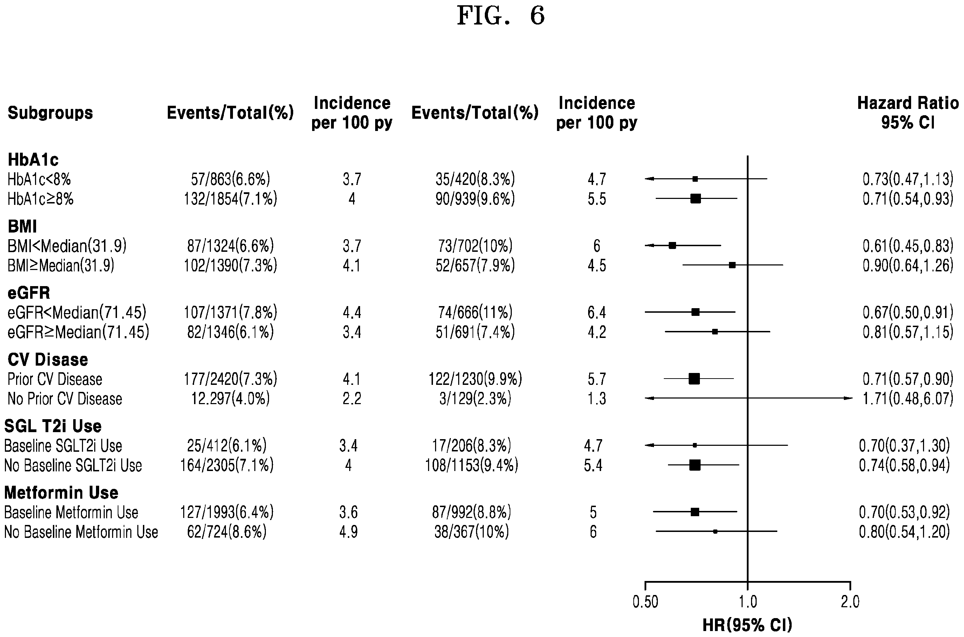

FIG. 5 and FIG. 6 show pre-specified subgroup analyses for the primary efficacy outcome.

**[Table 2]**

| | **Efpeglenatide administration group (4/6 mg)** | | **Placebo group** | | **HR (95 %CI)** | **P** | **P** |
|---|---|---|---|---|---|---|---|
| | **N (%)** | **N/100 py** | **N (%)** | **N/100 py** | | **(Superior ity)** | **(non-inferiority)** |
| Primary outcome (MACE) | 189 (7.0) | 3.9 | 125 (9.2) | 5.3 | 0.73 (0.58, 0.92) | 0.0069 | <0.0001 |
| Expanded MACE¹ | 257 (9.5) | 5.4 | 158 (11.6) | 6.8 | 0.79 (0.65, 0.96) | 0.020 | |
| Composite kidney outcome² | 353 (13.0) | 7.7 | 250 (18.4) | 11.6 | 0.68 (0.57, 0.79) | <0.0001 | |
| MACE or non-CV death | 216 (7.9) | 4.5 | 143 (10.5) | 6.0 | 0.73 (0.59, 0.91) | 0.0040 | |
| Renal function outcome³ | 121 (4.5) | 2.5 | 76 (5.6) | 3.1 | 0.77 (0.57, 1.02) | 0.072 | |
| MACE, non-CV death, HF, or renal function outcome | 243 (8.9) | 5.1 | 164 (12.1) | 7.0 | 0.71 (0.59, 0.87) | | |
| Myocardial Infarction | 91 (3.3) | 1.9 | 58 (4.3) | 2.4 | 0.75 (0.54, 1.05) | | |
| Non-fatal myocardial Infarction | 85 (3.1) | 1.7 | 53 (3.9) | 2.2 | 0.78 (0.55, 1.10) | | |
| Stroke | 47 (1.7) | 1.0 | 31 (2.3) | 1.3 | 0.74 (0.47, 1.17) | | |
| non-fatal Stroke | 41 (1.5) | 0.8 | 25 (1.8) | 1.0 | 0.80 (0.48, 1.31) | | |
| CV Mortality | 75 (2.8) | 1.5 | 50 (3.7) | 2.1 | 0.72 (0.50. 1.03) | | |
| Total Mortality | 111 (4.1) | 2.2 | 69 (5.1) | 2.8 | 0.78 (0.58, 1.06) | | |
| Coronary revascularization | 126 (4.6) | 2.6 | 66 (4.9) | 2.8 | 0.93 (0.69. 1.26) | | |
| Unstable angina | 6 (0.2) | 0.1 | 4 (0.3) | 0.2 | 0.55 (0.13, 2.35) | | |
| New macroalbuminuria | 348 (12.8) | 7.6 | 244 (18.0) | 11.3 | 0.68 (0.58, 0.80) | | |
| Heart Failure | 40 (1.5) | 0.8 | 31 (2.3) | 1.3 | 0.61 (0.38, 0.98) | | |
| Non-inferiority P values are for hazard ratios of 1.8 or less and 1.3 or less; | | | | | | | |
| MACE: major adverse CV event, defined as non-fatal myocardial infarction, non-fatal stroke, or death from cardiovascular (CV) causes; | | | | | | | |
| ¹ MACE or coronary revascularization or hospitalization for unstable angina; | | | | | | | |
| ² Incidentmacroalbuminuria (defined as urine albumin:creatinine greater than 33.9 mg/mmol and a 30 % or more increase from baseline, a 40 % or more decrease in eGFR of 30 days or more), renal replacement therapy for 90 days or more, or eGFR less than 15 ml/min/1.73 m² for 30 days or more); | | | | | | | |
| ³ Composite of either composite kidney outcome (with the exclusion of the albuminuria criteria) orall-cause death. | | | | | | | |

As shown in Table 2, 189/2717 (7.0 %) participants (3.9 per 100 person-years) in the 4 mg or 6 mg efpeglenatide administration group experienced at least one primary MACE outcome, compared with 125/1359 (9.2 %) participants (5.3 per 100 person-years) in the placebo group experienced at least one primary MACE outcome (HR 0.73, 95 % Cl 0.58-0.92; P non-inferiority less than 0.001 and P superiority 0.0069 at both 1.8 and 1.3 limits). As shown in FIG. 1 to FIG. 4, it was confirmed that the gap between the two groups was maintained until the end of the clinical trial.

Exploratory analysis suggested a possible dose-response effect, with a hazard ratio estimated at 0.82 (95 %CI 0.63, 1.06) and 0.65 (0.50, 0.86) for the 4 mg and 6 mg doses, respectively, compared to placebo.

The efpeglenatide administration group also had significantly lower incidence rate of at least one expanded MACE outcome (HR 0.79, 95 % Cl 0.65, 0.96; P=0.020), at least one composite kidney outcome (HR 0.68, 95 % Cl 0.57, 0.79; P < 0.0001), and at least one MACE or non-CV death (HR 0.73, 95 % Cl 0.59, 0.91; P ₌ 0.040) (FIG. 1 to FIG. 4).

Analyses of the impact of efpeglenatide on the primary outcome within predefined clinically important subgroups of participants showed no variation with sex, age, ancestry, duration of diabetes, HbA1c, body mass index (BMI), eGFR, history of prior CV disease, SGLT2 inhibitor use, or metformin use (FIG. 5 and FIG. 6).

Compared to placebo, the effect of efpeglenatide on the least square mean (LSM) difference in continuous variables during follow-up period included 1.24 % (95 %CI 1.17, 1.32) lower HbA1c, 0.9 kg/m² lower body mass index (95 %CI 0.8, 1.0), 2.6 kg (95 %CI 2.3, 2.9) lower body weight, each 1.5 mm Hg (95 %CI 0.8, 2.2) and 0. 6 mm Hg (95 %CI 0.2, 1.0) lower systolic and diastolic blood pressure, 2.1 mm Hg lower pulse pressure (95 %CI 1.4, 27), 3.9 bpm (95 %CI 3.4, 4.4) higher heart rate, 2. 7 mg/dl lower LDL cholesterol (0.07 mmol/l; 95 %CI 0.03, 0.12), 21 % lower UACR (95 %CI 14, 28), and 0.9 ml/min/1.73 m² (95 %CI 0.3, 1.5) higher LSM eGFR. An examination of the effect on changes in eGFR over time suggested a consistent effect during follow-up.

### Adverse effect

Specific adverse events of interest are shown in Table 3.

**[Table 3]**

| | **Efpeglenatide administration group** | **Placebo group** | **P** |
|---|---|---|---|
| | **N (%)** | **N (%)** | |
| Discontinuation for adverse events | 147 (5.4) | 49 (3.6) | 0.015 |
| Severe gastrointestinal events | 90 (3.3) | 25 (1.8) | 0.0090 |
| Constipation, diarrhea, nausea, vomiting, or bloating | 32 (1.2) | 6 (0.4) | 0.028 |
| Other severe gastrointestinal events | 59 (2.2) | 19 (1.4) | 0.10 |
| Confirmed pancreatic events | 32 (1.2) | 15 (1.1) | 0.87 |
| Confirmed pancreatic cancer | 2 (<0.1) | 3 (0.2) | N/A |
| Confirmed pancreatitis | 11 (0.4) | 7 (0.5) | 0.59 |
| Any cancer | 72 (2.6) | 37 (2.7) | 0.81 |
| Calcitonin rise > 5.9 pM (20 pg/ml) | 41 (1.5) | 20 (1.5) | 0.99 |
| Thyroid C-cell neoplasm | 0 | 0 | N/A |
| Severe hypoglycemia | 24 (0.9) | 13 (1.0) | 0.67 |
| Diabetic retinopathy and related complications | 47 (1.7) | 27 (2.0) | 0.50 |
| Acute renal failure | 88 (3.2) | 39 (2.9) | 0.62 |
| Severe injection site reaction | 0 | 1 (<0.1) | N/A |
| Severe allergic reaction | 5 (0.2) | 1 (<0.1) | N/A |
| Severe immune complex disease | 13 (0.5) | 1 (<0.1) | 0.096 |
| Efpeglenatide administration group data combines both the 4 mg and 6 mg dose administered groups; | | | |
| P values are from a Cox model for the relationship between random assignment to efpeglenatide and each adverse event adjusted for region and randomization stratum. | | | |

As shown in Table 3, more patients in the efpeglenatide administration group than in the placebo group (P=0.0090) reported severe gastrointestinal adverse effects, with the difference being limited to constipation, diarrhea, nausea, vomiting, or bloating (P=0.028). Other pre-specified safety outcomes (Table 3) and other adverse effects (Supplementary Tables S4 and S5) were similar in the two groups.

### Summary of results and conclusion

4076 participants were assigned to the efpeglenatide administration group (subject: 2717 people) or placebo group (subject: 1359 people). During a median follow-up period of 1.81 years, 189 (7.0 %) participants in the efpeglenatide administration group (3.9 per 100 person-years) and 125 (9.2 %) participants in the placebo group (5.3 per 100 person-years) experienced the primary outcome (HR 0.73, 95 % Cl 0.58, 0.92; P non-inferiority <0.001 and P superiority = 0.0069). The renal outcome (decreased renal or macroalbuminuria) affected 353 (13.0 %) participants in the efpeglenatide administration group and 250 (18.4 %) participants in the placebo group (HR 0.68, 95 %CI 0.57, 0.79; P < 0.0001). Diarrhea, constipation, nausea, vomiting, or bloating were more frequent in the efpeglenatide administration group.

In conclusion, weekly administration of 4 mg or 6 mg of efpeglenatide decreased the CV outcome and composite kidney outcome in type 2 diabetes mellitus patients with cardiovascular (CV) or renal disease and caused gastrointestinal adverse events.

### 3. Discussion

In this CV outcome trial, weekly subcutaneous injections of efpeglenatide (4 or 6 mg) reduced MACE by 27 % and reduced the composite kidney outcome by 32 % in patients with type 2 diabetes mellitus with CV or renal disease. These CV and renal benefits occured independently of baseline use of SGLT2 inhibitors, baseline use of metformin, and baseline eGFR. Moreover, they were associated with other GLP-1 RA typical gastrointestinal adverse events, but not with retinal, pancreatic or thyroid related events.

This trial shows that the previously reported salutary CV effects of GLP-1 RA on CV and renal outcomes extend to high-risk participants with a higher prevalence of renal disease than those included in the seven previously completed CV outcome trials of GLP-1 RA. Furthermore, the fact that these findings accrued with long-acting exendin-4-based GLP-1 RA suggests that the CV benefits of this class of agents are not restricted to GLP-1 RA based on human GLP-1. Finally, as more than 15% of participants concurrently used SGLT2i during the trial period and confidence intervals for the CV effect overlapped regardless of whether they were taking a SGLT2i inhibitor, this suggests that the beneficial CV effects of efpeglenatide may be independent of the effects of SGLT2 inhibitors.

Various possibilities may account for the cardioprotective effect and renal protective effect of efpeglenatide. Efpeglenatide significantly improved several risk factors for both CV and renal disease, including HbA1c, blood pressure, body mass index, LDL cholesterol, eGFR, and albuminuria. Indeed, published mediation analyses from CV outcomes trials of other GLP-1 RA have suggested that their CV and/or renal benefits may be partially mediated by their effects on HbA1c, blood pressure, or albuminuria, and a meta-regression analysis suggested a linear relationship between the degree of reduction in HbA1c using GLP-1 RA and the hazard of developing MACE.

As has been suggested for other GLP-1 RA, and by the observed reductions in composite kidney outcome, eGFR, and pulse pressure in this trial, efpeglenatide may also have salutary endothelial and microvascular effects. Other possible mechanisms include anti-inflammatory, anti-fibrotic, anti-atherosclerotic, and vasodilatory and other hemodynamic effects, as noted in studies of other GLP-1 RA.

The short follow-up period, fewer primary outcomes (314) than originally planned (330), and selection for previous CV and/or renal disease limit the power of the clinical trial and its relevance to low-risk patients with type 2 diabetes mellitus. Strengths include high medication adherence and follow-up, recruitment of a high-risk population for CV and renal outcomes, and inclusion of appreciable number of participants taking SGLT2 inhibitors. These features, plus the fact that a high proportion of overall participants were taking guideline-recommended cardioprotective and renal protective therapies, suggest that the results of this clinical trial may be generalizable to similar high-risk patients with type 2 diabetes mellitus.
The consistency of these results with the previous GLP-1 RA CV outcome trial clearly shows that efpeglenatide reduces severe CV and renal outcome in patients with type 2 diabetes mellitus and CV or renal disease.

## Claims

1. A pharmaceutical composition comprising a pharmaceutically effective amount of efpeglenatide and an excipient,
wherein the pharmaceutical composition prevents development of cardiovascular disease or renal dysfunction in a patient, or reduces the risk of development thereof.

2. The pharmaceutical composition of claim 1, wherein the patient is a type 2 diabetes mellitus patient.

3. The pharmaceutical composition of claim 1, wherein the patient is a patient with non-alcoholic steatohepatitis (NASH).

4. The pharmaceutical composition of claim 1, wherein the patient is a type 2 diabetes mellitus patient with HbA1c of greater than 7 %.

5. The pharmaceutical composition of claim 1, wherein the patient is a type 2 diabetes mellitus patient who has or is at risk of having a cardiovascular disease, renal disease, or both.

6. The pharmaceutical composition of claim 1, wherein the patient is a type 2 diabetes mellitus patient with a cardiovascular disease.

7. The pharmaceutical composition of claim 6, wherein the patient is a type 2 diabetes mellitus patient with cardiovascular disease, of 18 years of age or older.

8. The pharmaceutical composition of claim 6, wherein the cardiovascular disease is selected from coronary artery disease, stroke, and peripheral artery disease.

9. The pharmaceutical composition of claim 1, wherein the patient is a type 2 diabetes mellitus patient with a renal disease.

10. The pharmaceutical composition of claim 9, wherein the patient has an estimated glomerular filtration rate (eGFR) of 25 to 59.9 ml/min/1.73 m².

11. The pharmaceutical composition of claim 9, wherein the patient has one or more cardiovascular disease risk factors.

12. The pharmaceutical composition of claim 9, wherein the patient has one or more cardiovascular disease risk factors selected from (a) to (f) below:
(a) A body mass index (BMI) of 35 kg/m² or more;
(b) LDL of greater than 3.36 mmol/L and HDL of less than 1.03 mmol/L for men or HDL of less than 1.29 mmol/L for women, within the last 6 months while taking statins;
(c) smoking;
(d) a urine albumin-to-creatinine ratio (UACR) of 3 mg/mmol or more;
(e) systolic blood pressure of 140 mmHg or more and diastolic blood pressure of 90 mmHg or more, and taking antihypertensive medication; and
(f) first degree coronary artery disease at an age of less than 55 years for men or age of less than 65 years for women.

13. The pharmaceutical composition of claim 1, wherein the patient is a male of 50 years of age or older or a female of 55 years of age or older with type 2 diabetes mellitus, who has an estimated glomerular filtration rate (eGFR) of 25 to 59.9 ml/min/1.73 m² and one or more cardiovascular disease risk factors as defined in claim 12.

14. The pharmaceutical composition of claim 1, wherein the patient is a type 2 diabetes mellitus patient who has not used a glucagon-like peptide-1 (GLP-1) receptor agonist or a dipeptidyl peptidase-4 (DPP-4) inhibitor within the last 3 months.

15. The pharmaceutical composition of claim 1, wherein the efpeglenatide is to be administered subcutaneously.

16. The pharmaceutical composition of claim 1, wherein the efpeglenatide is administered weekly at 4 mg or 6 mg.

17. The pharmaceutical composition of claim 1, wherein the efpeglenatide is administered weekly at 2 mg for 4 weeks, and then administered weekly at 4 mg.

18. The pharmaceutical composition of claim 1, wherein the efpeglenatide is administered weekly at 2 mg for 4 weeks, then administered weekly at 4 mg for 4 weeks, and then administered weekly at 6 mg.

19. The pharmaceutical composition of claim 1, wherein the pharmaceutical composition is for treating type 2 diabetes mellitus in a patient at risk of developing cardiovascular disease or renal dysfunction.

20. The pharmaceutical composition of claim 1, wherein the cardiovascular disease is a major adverse CV event (MACE).

21. The pharmaceutical composition of claim 20, wherein the MACE is selected from non-fatal myocardial infarction, non-fatal stroke, and death from cardiovascular or unspecified causes.

22. The pharmaceutical composition of claim 1, wherein the pharmaceutical composition reduces the risk of developing a major adverse CV event (MACE) by 20 % or more compared to placebo.

23. The pharmaceutical composition of claim 1, wherein the renal dysfunction comprises one or more composite kidney outcomes selected from (a) to (d) below:
(a) Macroalbuminuria;
(b) a sustained decrease in eGFR of 40 % or more for 30 days or more;
(c) renal replacement therapy for 90 days or more; and
(d) eGFR of less than 15 ml/min/1.73 m² for 30 days or more continuously.

24. The pharmaceutical composition of claim 1, wherein the macroalbuminuria is defined as a UACR of greater than 33.9 mg/mmol and 30 % or more than baseline.

25. The pharmaceutical composition of claim 1, wherein the pharmaceutical composition reduces the risk of developing a composite kidney outcome as defined in claim 23 by 30 % or more compared to placebo.

26. The pharmaceutical composition of claim 1, wherein the cardiovascular disease comprises a major adverse CV event (MACE), coronary revascularization, and hospitalization for unstable angina.

27. The pharmaceutical composition of claim 1, wherein the pharmaceutical composition reduces the risk of developing death from a major adverse CV event (MACE) or non-cardiovascular death compared to placebo.

28. The pharmaceutical composition of claim 1, wherein the efpeglenatide is administered in combination with any one or more other therapeutic substances.

29. The pharmaceutical composition of claim 28, wherein the other therapeutic substances are selected from the group consisting of hypoglycemic drugs, cardioprotective drugs, anti-diabetic agents, anti-obesity agents, anti-hyperlipidemic agents, anti-atherosclerotic agents, anti-hypertensive agents, anti-platelet agents, anti-thrombotic agents and anti-coagulant agents.

30. The pharmaceutical composition of claim 28, wherein the other therapeutic substances are selected from sodium glucose co-transporter 2 (SGLT2) inhibitors and metformin.

31. A method of preventing development of cardiovascular disease or renal dysfunction in a patient with type 2 diabetes mellitus, or reducing the risk of development thereof, comprising administering an effective amount of efpeglenatide to the patient.

32. A method of treating type 2 diabetes mellitus, comprising administering an effective amount of efpeglenatide to a patient,
wherein the patient does not experience adverse effects associated with cardiovascular disease or renal dysfunction during treatment.

33. The method of claim 31 or 32, wherein the patient is a type 2 diabetes mellitus patient with HbA1c of greater than 7 %.

34. The method of claim 31 or 32, wherein the patient is a type 2 diabetes mellitus patient who has or is at risk of having cardiovascular disease, renal disease, or both.

35. The method of claim 31 or 32, wherein the patient is a type 2 diabetes mellitus patient with cardiovascular disease.

36. The method of claim 31 or 32, wherein the patient is a type 2 diabetes mellitus patient with cardiovascular disease, of 18 years of age or older.

37. The method of claim 36, wherein the cardiovascular disease is selected from coronary artery disease, stroke, and peripheral artery disease.

38. The method of claim 31 or 32, wherein the patient is a type 2 diabetes mellitus patient with renal disease.

39. The method of claim 38, wherein the patient has an estimated glomerular filtration rate (eGFR) of 25 to 59.9 ml/min/1.73 m².

40. The method of claim 38, wherein the patient has one or more cardiovascular disease risk factors.

41. The method of claim 38, wherein the patient has one or more cardiovascular disease risk factors selected from the following (a) to (f):
(a) A body mass index (BMI) of 35 kg/m² or more;
(b) LDL of greater than 3.36 mmol/L and HDL of less than 1.03 mmol/L for men or HDL of less than 1.29 mmol/L for women, within the last 6 months while taking statins;
(c) smoking;
(d) a urine albumin-to-creatinine ratio (UACR) of 3 mg/mmol or more;
(e) systolic blood pressure of 140 mmHg or more and diastolic blood pressure of 90 mmHg or more, and taking antihypertensive medication; and
(f) first degree coronary artery disease at an age of less than 55 years for men or an age of less than 65 years for women.

42. The method of claim 31 or 32, wherein the patient is a male of 50 years of age or older or a female of 55 years of age or older with type 2 diabetes mellitus who has an estimated glomerular filtration rate (eGFR) of 25 to 59.9 ml/min/1.73 m² and one or more cardiovascular disease risk factors as defined in claim 12.

43. The method of claim 31 or 32, wherein the patient is a type 2 diabetes mellitus patient who has not used a glucagon-like peptide-1 (GLP-1) receptor agonist or a dipeptidyl peptidase-4 (DPP-4) inhibitor within the last 3 months.

44. The method of claim 31 or 32, further comprising identifying, prior to administration of efpeglenatide, a patient who has any of the following:
(1) A type 2 diabetes mellitus patient with coronary artery disease, stroke, or peripheral artery disease; or
(2) a type 2 diabetes mellitus patient who has an eGFR of 25 to 59.9 ml/min/1.73 m² and meets one or more of the following cardiovascular disease risk factors (a) to (f):
(a) A body mass index (BMI) of 35 kg/m² or more;
(b) LDL of greater than 3.36 mmol/L and HDL of less than 1.03 mmol/L for men or HDL of less than 1.29 mmol/L for women within the last 6 months while taking statins;
(c) smoking;
(d) a urine albumin-to-creatinine ratio (UACR) of 3 mg/mmol or more;
(e) systolic blood pressure of 140 mmHg or more and diastolic blood pressure of 90 mmHg or more, and taking antihypertensive medication; and
(f) first degree coronary artery disease at an age of less than 55 years for men or an age of less than 65 years for women.

45. The method of claim 31 or 32, wherein the administration is subcutaneous administration.

46. The method of claim 31 or 32, wherein the effective amount of efpeglenatide is 4 mg or 6 mg.

47. The method of claim 31 or 32, wherein the efpeglenatide is administered weekly at 4 mg or 6 mg.

48. The method of claim 31 or 32, wherein the efpeglenatide is administered weekly at 2 mg for 4 weeks, then administered weekly at 4 mg.

49. The method of claim 31 or 32, wherein the efpeglenatide is administered weekly at 2 mg for 4 weeks, then administered weekly at 4 mg for 4 weeks, and then administered weekly at 6 mg.

50. The method of claim 31 or 32, wherein the cardiovascular disease is a major adverse CV event (MACE).

51. The method of claim 50, wherein the MACE is selected from non-fatal myocardial infarction, non-fatal stroke, and death from cardiovascular or unspecified causes.

52. The method of claim 31 or 32, wherein the efpeglenatide reduces the risk of a major adverse CV event (MACE) development by 20 % or more compared to placebo.

53. The method of claim 31 or 32, wherein the adverse effects associated with renal disease or renal dysfunction comprise one or more composite kidney outcomes selected from (a) to (d) below:
(a) Macroalbuminuria;
(b) a sustained decrease in eGFR of 40 % or more for 30 days or more;
(c) renal replacement therapy for 90 days or more; and
(d) eGFR of less than 15 ml/min/1.73 m² for 30 days or more continuously.

54. The method of claim 53, wherein the macroalbuminuria is defined as a UACR greater than 33.9 mg/mmol and 30 % or more than baseline.

55. The method of claim 31 or 32, wherein the efpeglenatide reduces the risk of developing a composite kidney outcome as defined in claim 53 by 30 % or more compared to placebo.

56. The method of claim 31 or 32, wherein the cardiovascular disease comprises a major adverse CV event (MACE), coronary revascularization, and hospitalization for unstable angina.

57. The method of claim 31 or 32, wherein the efpeglenatide reduces the risk of developing death from a major adverse CV event (MACE) or non-cardiovascular death compared to placebo.

58. The method of claim 31 or 32, wherein the efpeglenatide is administered in combination with any one or more other therapeutic substances.

59. The method of claim 58, wherein the other therapeutic substances are selected from the group consisting of hypoglycemic drugs, cardioprotective drugs, anti-diabetic agents, anti-obesity agents, anti-hyperlipidemic agents, anti-atherosclerotic agents, anti-hypertensive agents, anti-platelet agents, anti-thrombotic agents and anti-coagulant agents.

60. The method of claim 58, wherein the other therapeutic substances are selected from sodium glucose co-transporter 2 (SGLT2) inhibitors and metformin.

61. A use of efpeglenatide for use in treatment of a patient with type 2 diabetes mellitus,
wherein the efpeglenatide prevents development of cardiovascular disease or renal dysfunction in a patient, or reduces the risk of development thereof.
